# EUROPEAN PATENT APPLICATION

(11) **EP 4 242 647 A1**
(43) Date of publication of application: **13.09.2023**
(21) Application number: 22160921.7
(22) Date of filing: 08.03.2022
(51) Int. Cl.: G01N 27/12, G01N 33/00, H04W 4/38

(54) **DEVICE AND METHOD FOR DETERMINING A CONCENTRATION OF A TARGET GAS IN A MOBILE APPLICATION**

(71) Applicant: Infineon Technologies AG, 85579 Neubiberg (DE)
(72) Inventor: SCHOBER, Sebastian, 80469 München (DE); CARBONELLI, Cecilia, 81477 München (DE)
(74) Representative: Hersina, Günter

(57) **Abstract**

A device for determining a concentration of a target gas in a mobile application comprises: a measurement module configured for obtaining measurement information about a concentration measurement of a target gas; a communication module configured for communicating with at least one further device via a direct wireless communication path, wherein the communication module is configured for receiving information about a further concentration measurement of the target gas from the further device; and a calibration module configured for using calibration information for determining a calibrated measurement value on the basis of the measurement information, and configured for revising the calibration information by using the received information about the further concentration measurement.

## Description

Examples of the present disclosure relate to a device for determining a concentration of a target gas in a mobile application. For example, the device comprises, or is part of, a gas sensing device and/or a mobile apparatus such as a mobile user device. Further examples of the present disclosure relate to a method for determining a concentration of a target gas in a mobile application. Some examples relate to an internet of things (IOT)-based technique for blind drift calibration of mobile environmental sensor devices.

Gas sensors, for example chemo resistive gas sensors, may provide a small sized and low cost solution for assessing air quality. In particular, these gas sensors may be integrated in portable devices, such as smartphones or wearable. However, chemo resistive sensors often experience a drift behavior of the sensor signal over longer time scales. This effect may continuously worsen the prediction quality of these sensors, e.g., the accuracy of a calibrated measurement value determined from the sensor signal. Therefore, recalibration or drift compensation techniques are required in order to maintain a good measurement accuracy. In mobile applications, however, many of these techniques are not feasible, for example, because a reference sensor providing a true value of the gas concentration to be determined may not be available. An overview over different drift calibration methods is given by F. Delaine, B. Lebental and H. Rivano in "In Situ Calibration Algorithms for Environmental Sensor Networks: A Review," IEEE Sensors Journal, vol. 19, no. 15, pp. 5968-5978, 1 Aug.1, 2019, doi: 10.1109/JSEN.2019.2910317. Existing approaches address various sensor scenarios, also including mobile sensors in blind or semi-blind scenarios, i.e., the sensors have no or limited access to reference information which may be used for calibration.

However, in view of the state of the art, it would be desirable to have a concept for determining a concentration of a target gas in a mobile application, which concept provides an improved tradeoff between a high accuracy of the determined concentration, a large extent of independency, e.g., from a base station or a centralized network or the availability of reference information, and a low requirement of hardware resources such as computational power or data storage.

Examples of the present disclosure rely on the idea to revise, e.g., check and/or adapt, calibration information, which is used for determining a calibrated measurement value on the basis of measurement information related to the concentration of a target gas, on the basis of measurement information determined by multiple mobile devices, which directly communicate with each other via a wireless communication path. For example, a mobile device may directly communicate information about one or more measurements of the concentration to one or more further mobile devices, which are located, at least temporarily, in the proximity of the mobile device. In turn, the mobile device may receive information about one or more measurements of the concentration performed by one or more of the further mobile devices. Due to the proximity of the mobile devices, the mobile device may assume correspondence, or at least similarity, between concentrations of the target gas determined by the mobile device and the one or more further mobile devices. In other words, examples of the present disclosure may exploit information about the concentration of a target gas gathered by multiple mobile devices directly communicating with each other for revising the calibration of one of the mobile devices. This concept may allow for calibrating the mobile device without the need of a higher level instance, such as a server as in cloud based solutions, and without the need of availability of a reference sensor, and may thus provide for an accurate determination of a calibrated measurement value of the concentration, e.g., in blind scenarios.

Examples according to the present disclosure provide a device for determining a concentration of a target gas in a mobile application. For example, the device may be part of a mobile apparatus or a mobile device. The device comprises a measurement module configured for obtaining measurement information about a concentration measurement of a target gas. Further, the device comprises a communication module configured for communicating with at least one further device via a direct wireless communication path. The communication module is further configured for receiving information about a further concentration measurement of the target gas from the further device. The device further comprises a calibration module configured for using calibration information for determining a calibrated measurement value on the basis of the measurement information. Further, the calibration module is configured for revising the calibration information by using the received information about the further concentration measurement.

Further examples of the present disclosure provide a method for determining a concentration of a target gas in a mobile application. The method comprises a step of obtaining measurement information about a concentration measurement of the target gas.

The method further comprises a step of receiving information about a further concentration measurement of the target gas via a direct wireless communication interface. Further, the method comprises a step of using calibration information for determining a calibrated measurement value on the basis of the measurement information. The method comprises a step of revising the calibration information by using the received information about the further concentration measurement.

Advantageous implementations are defined in the dependent claims.

Examples of the present disclosure are described in more detail below with respect to the figures, among which:
- Fig. 1A, B: illustrate a plurality of interacting mobile devices according to an example,
- Fig. 2: illustrates a schematic block diagram of a device and a method for determining a concentration of a target gas according to an example,
- Fig. 3: illustrates an example of a mobile apparatus,
- Fig. 4: illustrates an example of a data gathering phase,
- Fig. 5: illustrates an example of a data exchange between two mobile devices,
- Fig. 6: illustrates an example of an operation scheme for revising calibration information,
- Fig. 7: illustrates an example of a gas sensing device,
- Fig. 8A: illustrates examples of simulated concentration profiles,
- Fig. 8B: illustrates an exemplary comparison of exchanged and measured data with true concentration values,
- Fig. 9: illustrates an exemplary comparison between true and predicted offsets.

In the following, examples are discussed in detail, however, it should be appreciated that the examples provide many applicable concepts that can be embodied in a wide variety of sensor calibration applications. The specific examples discussed are merely illustrative of specific ways to implement and use the present concept, and do not limit the scope of the examples. In the following description, a plurality of details is set forth to provide a more thorough explanation of examples of the disclosure. However, it will be apparent to one skilled in the art that other examples may be practiced without these specific details. In other instances, well-known structures and devices are shown in form of a block diagram rather than in detail in order to avoid obscuring examples described herein. In addition, features of the different examples described herein may be combined with each other, unless specifically noted otherwise.

In the following description of examples, the same or similar elements or elements that have the same functionality are provided with the same reference sign or are identified with the same name, and a repeated description of elements provided with the same reference number or being identified with the same name is typically omitted. Hence, descriptions provided for elements having the same or similar reference numbers or being identified with the same names are mutually exchangeable or may be applied to one another in the different examples.

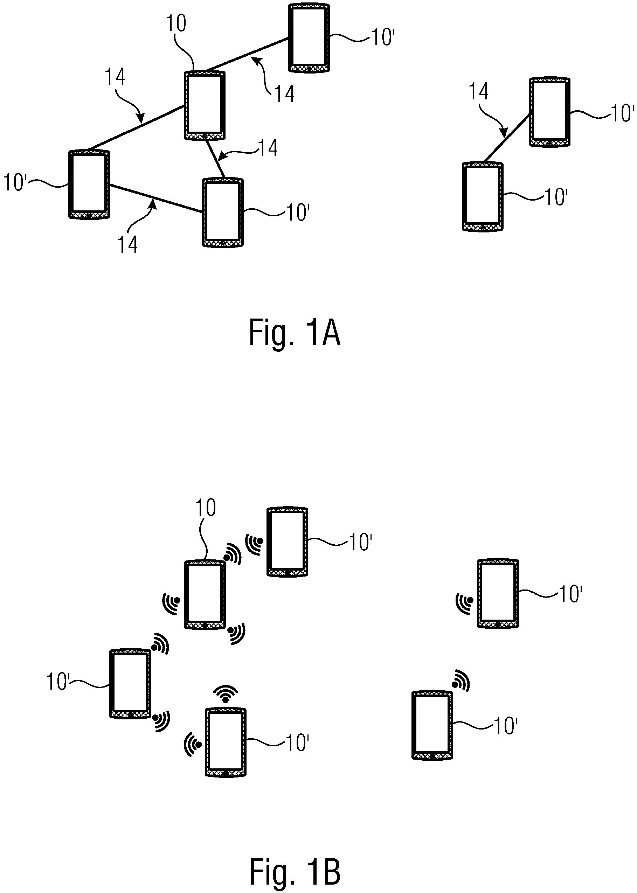

Fig. 1a illustrates a plurality of mobile apparatuses 10, also referred to as mobile devices 10, according to an application scenario of the present disclosure. Each of the mobile devices 10 may be configured for monitoring a parameter concerning the air quality within the surroundings of the respective mobile device. To this end, the mobile device is configured for determining the concentration of at least one target gas. For descriptive purpose, one of the mobile devices is referenced using reference signal 10, while the further mobile devices are referenced using reference sign 10'. It is, however, noted that the description of the mobile device 10 may apply to any other of the mobile devices. Accordingly, the mobile device 10 and the further mobile device 10' may be of the same type, or may be all be configured for performing the herein disclosed concept for determining the gas concentration and revising its calibration information. For example, the mobile devices 10, 10' may represent nodes of a wireless sensor network, or as sesor loT nodes. The mobile devices may be non-stationary devices, for example, the mobile devices may be smartphones or wearable devices.

While the mobile devices 10, 10' are moving in space, the mobile device 10 may get proximate to one or more of the further mobile devices 10'. In Fig. 1a, the distance between the mobile devices 10, 10' is indicated using reference sign 14. Mobile devices close to each other may be able to communicate with each other via a direct wireless communication interface, such as Bluetooth, as illustrated in Fig. 1b. The wireless communication interface used for communication between the mobile devices 10, 10' may provide for a wireless connection between two of the mobile devices if the distance 14 between the two mobile devices is below a threshold or, in other words, within a range of the wireless communication interface. In examples, the range of the wireless communication interface may depend on an operation mode and may, therefore, be controlled by selecting a specific operation mode. A direct wireless communication path between two devices may refer to a communication path in which the wireless part of the path does not comprise a further device such as a base station or server.

Accordingly, the mobile device 10 may scan for further mobile devices 10' being located within the range of the wireless communication interface, and may communicate with the further mobile devices 10' being within the range.

For example, the mobile devices 10, 10' of Fig. 1a, 1b may be, or may be configured as, air quality index (AQI) measurement systems.

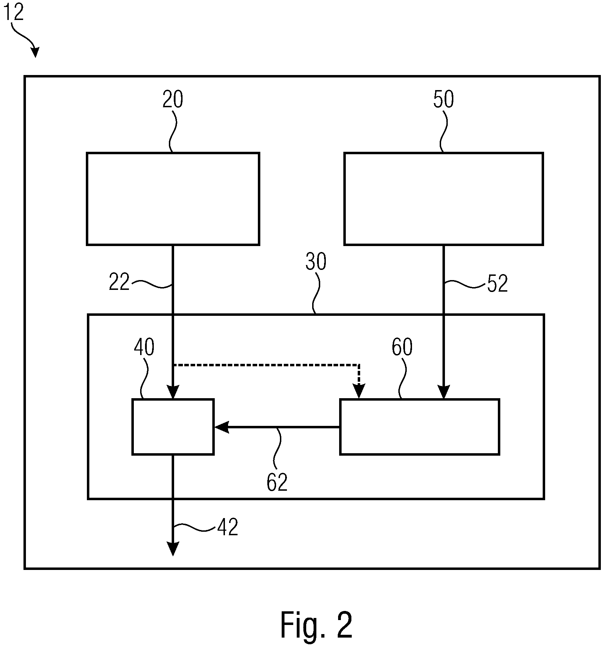

Fig. 2 illustrates a schematic block diagram of a device 12 for determining a concentration of a target gas in a mobile application according to an example. For example, the device 12 may be for use in a mobile device, such as mobile device 10. The device 12 may be part of the mobile device 10, or may be implemented by means of the mobile device 10. The device 12 comprises a measurement module 20, which is configured for obtaining measurement information 22 about a concentration measurement of a target gas. In examples, the measurement module 20 may determine the measurement information, e.g., by measuring a sensor signal of a gas sensing device (also referred to as gas sensing unit), which may be part of the measurement module 20. In alternative examples, the measurement module 20 may receive the measurement information, e.g. from a gas sensing device. The device 12 further comprises a communication module 50, which is configured for communicating with at least one further device via a direct wireless communication path. For example, the further device is part of a further mobile apparatus, e.g. one of the further mobile devices 10' of Fig. 1, or may be implemented by means of one of the further mobile devices 10'. A communication module 50 is configured for receiving information about a further concentration measurement of the target gas from the further device. For example, a mobile device 10, which comprises the device 12, may be configured for communicating with the further mobile device 10', which comprises the further device, via the direct wireless communication path to receive the information about the further concentration measurement. The device 12 further comprises a calibration module 30. Calibration module 30 comprises a signal calibration unit 40 configured for using calibration information 62 for determining a calibrated measurement value 42 on the basis of the measurement information 22. The calibration module 30 further comprises a calibration model unit 60 configured for revising the calibration information 62 by using the received information 52 about the further concentration measurement.

For example, the calibrated measurement value 42 represents a prediction or an estimation of a true value of the concentration of the target gas. The signal calibration unit 40 may determine the calibrated measurement value 42 by applying a calibration model, which depends on the calibration information 62, to the measurement information 22. In other words, the calibration information 62 may comprise one or more calibration parameters.

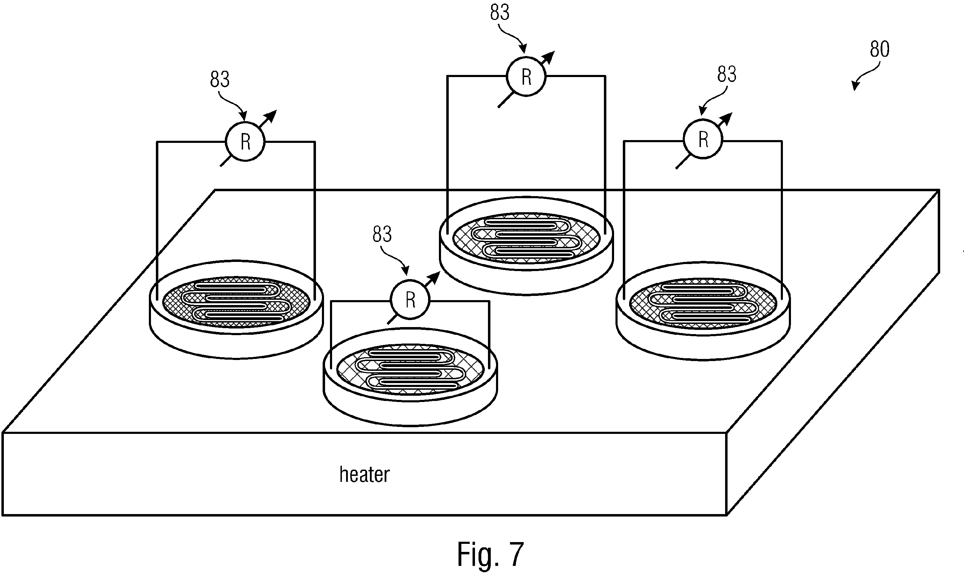

The measurement information 22 may, for example, include one or more values of one or more measurement signals provided by a gas sensing device, for example a gas sensing device as described with respect to Fig. 7 below. For example, the measurement information 22 may be provided by a chemoresistive gas sensing device. For example, For example, the target gas may be Ozone or CO₂.

Gas sensing devices, in particular chemoresistive gas sensing devices, may experience drift over time. Drift can manifest itself in different ways. In some cases only a baseline value (e.g. a resistance value of a chemoresistive gas sensing device in the absence of polluting gases) changes. In other, more extreme scenarios, also the sensitivity and the response of the sensor to different concentrations can vary over time. In this case, the calibration curve (or model) needs to be adjusted or even obtained from scratch. Examples of the present disclosure address the drift problem for the specific case of mobile and connected gas sensors by implementing a scheme for blind drift calibration, e.g, with continuous or repeated data exchange between mobile sensors within in short distance ranges.

For example, the device 12 may be implemented by means of a signal processor, or by means of an application running on a signal processor. In other words, the measurement module 20, the communication module 50 and the calibration module 30 may, in examples, be regarded as parts of an operation scheme of a signal processor. In this regard, the measurement module 20 may be regarded as an interface for receiving the measurement information. In alternative examples, the measurement module 20 may comprise a gas sensing device for determining the measurement information 22. The communication module 50 may be regarded as an interface for receiving the information 52 about the further concentration measurement. A wireless communication interface, which may be part of the wireless communication path, or which may provide the wireless communication path, may in examples be part of the device 12, e.g. of the communication module. Alternatively, the wireless communication interface may be part of a mobile device, which comprises the device 12, that is, device 12 may use the wireless communication interface of a mobile device 10.

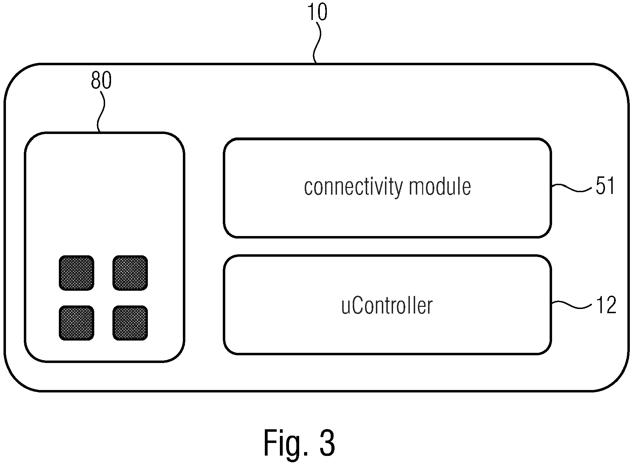

Fig. 3 illustrates an example of a mobile apparatus 10 according to an example of the present disclosure. According to the example of Fig. 3, the mobile apparatus 10 comprises the device 12, and further comprises a gas sensing device 80 and a connectivity module 51. The connectivity module 51 comprises a wireless communication interface as part of the wireless communication path, using which the device 12 communicates with in the further device. For example, the gas sensing device 80 may be implemented by means of a sensor array, for example as described with respect to Fig. 7.

In an alternative implementation of the mobile apparatus 10, the gas sensing device 80 may be part of the device 12 and/or the connectivity module 51 may be part of the device 12 of the mobile apparatus 10, as mentioned above. Accordingly, as described with respect to Fig. 2 and Fig. 3, device 12 may be implemented merely by means of a signal processor, or may optionally comprise one or both of a sensing device and a wireless communication interface.

It is noted that the schematic block diagram of Fig. 2 also serves for illustration of a method for determining a concentration of a target gas, in which the modules 20, 30, 50 represent steps of the method. Accordingly, the description of the device 12 may be understood of both a device and a method for determining a concentration of a target gas.

In the following, the description of Fig. 2 is continued, details of which may also apply to the mobile device 10 of Fig. 3. It is further pointed out that the mobile device 10 may represent the mobile device 10 of Fig. 1a and Fig. 1b.

In other words, Fig. 3 illustrates a possible high level architecture of the mobile device 10, which may represent a sensor node of a wireless sensor network. The components of the mobile device 10 may comprise a sensor array 80 interacting with the air and the gasses to be analyzed, a microcontroller module, e.g., provided by device 12, for the conditioning and signal processing of the sensor raw data, and a connectivity module 51, which may ensure the connectivity between different mobile sensors.

By using the information 52 about the further concentration measurement received from the further device of a further mobile device 10', the herein disclosed concept allows for a blind calibration process of environmental sensors, for example, in an IOT scenario. The term blind calibration may be understood so that no calibration against a reference gas analyzer is required. Accordingly, the herein disclosed concept provides a cost efficient and time saving way of revising the calibration model used for calibrating the measurement information 22. For example, merely data collected from the own sensing unit, e.g., data obtained by the measurement module 20, and information provided by the further mobile devices 10' may be used for revising the calibration information 62, for example to adjust the gas predictions.

Accordingly, examples of the present disclosure rely on the idea to allow mobile sensor devices or mobile devices functioning as sensor devices, to exchange measurement data, or information obtained from measurement data, with other sensor devices, if they are close enough to each other to exchange data via the wireless communication interface, or if they are within a distance range of each other, which is below a specific threshold. The criterion on the distance, or the pure fact that the devices are within the range of the wireless communication interface, may ensure that the concentration of the target gas, which both devices are exposed to, is similar enough to allow a comparison between both measurements, e.g., by assuming that both concentrations are equal or similar.

For example, the device 12 may be employed in a IOT scenario to track ground level pollution and in real life environment where ambient conditions may affect the behavior of sensing components. Due to the ability to recalibrate the sensing device on the edge using the herein disclosed concept, low cost components may be employed which may be subject to a higher risk of sensor drift. Further, the disclosed approach may allow for monitoring the performance of the sensing devices over time and over various locations.

According to examples, the wireless communication path is provided via Bluetooth. As Bluetooth has a limited range, the device 12 may assume from the pure fact that a connection to the further device may be established that the further device is within a range of the device 12, within which a range the concentration measurement underlying the measurement information 22 and the further concentration measurement by the further device may be assumed to refer to a similar concentration of the target gas.

According to examples, the device 12 may estimate a distance between the mobile device 10 and the further mobile device 10' on the basis of a received signal strength of a signal provided by the further mobile device 10' via the wireless communication path. Using the signal strength may provide for an accurate estimation of the distance between the mobile device 10 and the further mobile device 10' and may therefore provide an accurate assessment whether or not the mobile device 10 and the further mobile device 10' are exposed to a similar concentration of the target gas.

As the revising of the calibration information 62 is performed by the device 12, the revising of the calibration information 62 is performed on the edge, in contrast to cloud-based approaches that process sensor data on a server based application and distribute the calibration to sensor devices of a network. Accordingly, examples of the present disclosure may be independent of the availability of a server. In contrast, examples of the present disclosure may exploit concentration measurements of any further mobile devices capable of sensing the target gas and communicating information about their concentration measurements. For example, device 12 may collect a variety of calibration related data, which may include a quantification of the information quality of the collected data, and device 12 may translate the collected data into a drift estimation. The collected data may further include sensor specific characteristics, allowing for an easy revising of the calibration information.

According to examples, the information 52 about the further concentration measurement comprises an indication of a further calibrated measurement value of the concentration of the target gas, the further calibrated measurement value being determined by the further device. Additionally or alternatively, the information 52 may comprise an indication of further measurement information obtained by the further device. For example, the further measurement information may include raw data of a measurement signal. The further calibrated measurement data may, for example, be determined by the further device as described with respect to device 12 for the calibrated measurement value 42. For example, the further calibrated measurement value and/or the indication of further measurement information signaled in the information 52 may relate to a most recent measurement of the concentration performed by the further mobile device 10' or determined by the further device. Accordingly, it may be assumed that the transmitted information relates to a time instance, at which the mobile device 10 and the further mobile device 10' are close to each other.

According to examples, the information 52 about the further concentration measurements further comprises an indication of a time instance to which the further concentration measurement refers. Transmitting the time instance allows for associating the information about the further concentration measurement to measurement information 22 obtained by the mobile device 10 at a time instance which is close to the time instance to which the further concentration measurement refers.

According to examples, the calibration revising unit 60, also referred to as recalibration unit 60, uses, for revising the calibration information 62, information about a confidence of the further concentration measurement, or the information 52 about the further concentration measurement, and/or information about a confidence of the calibrated measurement value 42 or the measurement information 22.

Accordingly, in examples, the information 52 about the further concentration measurement comprises an indication of a confidence of the further concentration measurement, and the calibration module 30 determines a contribution of the information 52 about the further concentration measurement for revising the calibration information 62 on the basis of the indication of the confidence of the further concentration measurement. For example, if the indication of the confidence of the further concentration measurement indicates a low confidence of the further concentration measurement, the calibration module 30 may refrain from using an indication of a concentration of the target gas comprised in the received information 52 for revising the calibration information 62, or may set a contribution of the received information 52 on the revising of the calibration information 62 to be low.

According to examples, device 12 is configured for determining an indication of a confidence of the calibrated measurement value 42. Device 12 may consider the confidence of the concentration measurement value 42 for revising the calibration information 62. For example, the calibration module 30 may compare a confidence of the calibrated measurement value 42 with a confidence of the further concentration measurement received from the further device, and may weight respective contributions in revising the calibration information 52 according to their respective confidences.

According to examples, device 12 may determine the indication of the confidence of the calibrated measurement value 42 by using one or more of a temporal evolution of a sequence of concentration measurements, an age of a sensing unit, which provides the measurement information, an information about a functional state of the sensing unit, and a time instance of a previous revision of the calibration information 62. Equivalently, the further device may use one or more of these criterions for determining the confidence of the further concentration measurement.

For example, using the temporal evolution of the sequence of concentration measurements may rely on the assumption that the concentration of a specific gas takes a specific value from time to time. For example, for some gases, e.g. Ozone, it may be assumed that the concentration reaches a value close to zero at least once a day. Accordingly, based on the finding that local minima of the concentration determined from a temporal sequence of concentration measurements shift over time may be a hint towards a sensor drift, and the device 12 may accordingly choose a lower confidence compared to a scenario in which the local minima do not shift over time.

In examples, the confidence for the measurement information 22 and/or the information 52 about the further concentration measurement may be determined using a history about an exposure of the respective sensing device, i.e. mobile device 10 or mobile device 10', to a specific gas, which may be the target gas or a further target gas. For example, the specific gas may be ozone, which may lead to a particularly high sensor drift, so that a high exposure may be an indication of a low confidence. Accordingly, in examples, in which the target gas is not the specific gas, the information 52 about the further concentration measurement may further comprise, in addition to the information about the history regarding the target gas, information about a minimum and/or maximum concentration of a further specific gas.

As mentioned before, the measurement information 22 may comprise one or more measurement values of a measurement signal measured by one or more sensing units. Gas sensing devices, in particular chemo resistive gas sensing devices, rely on the principle that the presence of gas molecules of a target gas of the gas sensing device at a sensing surface region of the sensing device cause a change of a resistance of a sensing material, which resistance is measured so as to obtain the measurement signal. The measurement signal may, for example, drift over time as gas molecules of the target gas or further gases present in the air may accumulate at the sensing surface region. Other reasons for sensor drift may be aging of the sensing material. For example, the measurement signal of a sensing device such as a chemo resistive sensing device may be characterized by a base line value, which may, for example, characterize a magnitude of the sensor signal at a specific concentration of the target gas, e.g., zero. Additionally, the measurement signal may be characterized by a sensitivity of the sensing device, e.g., a change of the measurement signal with respect to a change of the concentration, which may be expressed by a slope value. Both the base line value, also referred to as offset value, and the sensitivity, or slope value, may drift over time.

For example, calibration of the measurement information 22, i.e., the determination of the calibrated measurement value 42 on the basis of the measurement information 22, may be performed by offsetting a measurement value comprised in the measurement information 22 using the base line value, for example, by adding or subtracting the baseline value from the measurement value. Additionally or alternatively, the calibration of the measurement information may comprise a scaling of the measurement value using a slope value. The baseline value and/or the slope value may optionally be part of the calibration information 62, and may be subject to revising the calibration information.

The calibration of the measurement information 22 may comprise, additionally or alternatively to the above mentioned usage of a baseline value and/or slope value, using a machine learning model, e.g. a neural network, for determining the calibrated measurement value 42 on the basis of the measurement information 22. Using a machine learning model allows for considering the temporal evolution of the measurement information 22, e.g., by means of a recurrent neural network. According to examples, the calibration information 62 may comprise parameters, such as weights, of the machine learning model, e.g., the neural network used for calibrating the measurement information 22. For example, in case that a drift of the sensitivity or slope value is detected, the parameters of the machine learning model may be scaled in accordance with the change of sensitivity. Additionally or alternatively, the measurement information 22 may be scaled in dependence on the calibration information 62 prior to being processed by the machine learning model. Accordingly, the calibration information 62 may comprise one or two or all of a base line value, a slope value, and a parameter set for a machine learning model used by the signal calibration unit 40 for calibrating the measurement information 22.

In the following, various approaches for revising the calibration information 62 are described. Revising the calibration information 62 may be performed by, for example, checking whether or not a response of a sensing unit providing the measurement information 22 has changed since a preceding determination or revising of the calibration information 62, and if so, revising the calibration information 62 may comprise adapting the calibration information. Additionally or alternatively, revising the calibration information 62 may be performed by comparing one or more calibrated measurement values 42 with information 52 about further calibration measurements performed by one or more further devices and, in dependence on the outcome of the comparison, keeping the calibration information 62 or adopting the calibration information 62 according to the outcome of the comparison.

According to examples, the calibration module 30 uses information about a plurality of further concentration measurements received from a plurality of further devices, which may, for example, be part of the further mobile devices 10' during a time interval for revising the calibration information 62.

For example, the device 12 may gather information about concentration measurements from a plurality of further devices during the time interval, wherein, the information about each of the further concentration measurements may include an information about a time instance to which the respective further concentration measurement refers. For example, after the time interval of gathering information, the device 12 may perform the revising of the calibration information 62 using the information about the further concentration measurements gathered during the time interval. Having a high number of concentration measurements available performed by various different further mobile sensing devices may provide for an accurate recalibration because uncertainties in the gathered information may be eliminated due to statistics on a high number of measurements. In other words, according to examples, the exchanged information is locally stored over a long time frame before undertaking the calibration step.

According to examples, the time interval, during which the device 12 gathers information on further concentration measurements, is between one hour and one week, or between four hours and one week, or between twelve hours and three days, or between twelve hours and one day.

According to examples, the length of the time interval depends on a number on encounters with further devices providing information on further concentration measurements. Additionally or alternatively, the time interval may depend on the confidence of the received information about the further concentration measurements.

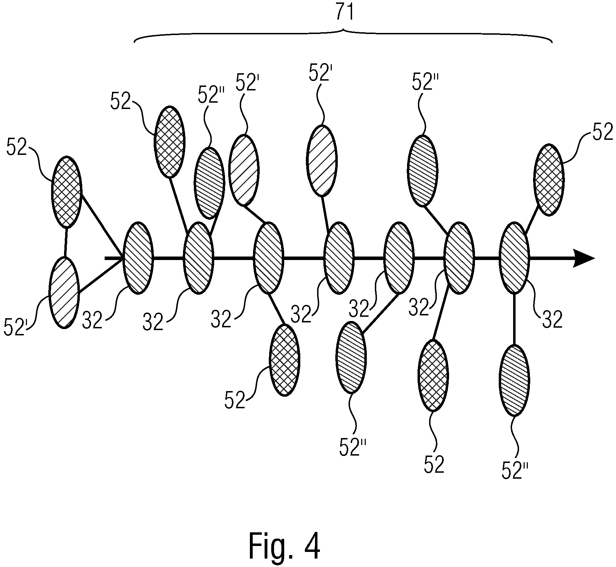

Fig. 4 illustrates a time interval 71 during which data for revising the calibration information 62 is gathered. Device 12 buffers for multiple time instances during the data gathering interval 71 measurement information 22 and/or calibrated measurement values 42 determined from the measurement information 22 of the respective time instances. Information obtained by device 12 itself is indicated using reference sign 32 in Fig. 4. Accordingly, in Fig. 4, nodes 32 represent the device which is to be evaluated. Further, device 12 receives and buffers information about further concentration measurements determined by further devices which get within a communication range or a specific distance to device 12 during time interval 71. In the temporal graph structure of Fig. 4, the connection between the nodes represent an information exchange between the sensors. In the exemplary scenario of Fig. 4, device 12 has multiple contacts with a first further device providing information 52, multiple encounters with a second further device providing information 52', and multiple encounters with a third further device providing information 52". Recalibration unit 60 may use information 30, 52, 52', 52" for revising the calibration information 62, wherein the recalibration unit 60 may optionally determine respective contributions of the information received from the further devices and information 32 based on respective confidences and/or respective positions of the devices or distances of the devices associated with the information received from the individual encounters.

The information gathered during the time interval 71 may subsequently be used for recalibration 60, e.g. by determining or predicting a measurement offset or compensation factor.

As mentioned before, the calibration information 62 may comprise a base line value, the baseline value being indicative of a constant calibration offset between a measurement value of the measurement information 22 and the calibrated measurement value, which calibration offset may, for example, be applied to the measurement information 22 for obtaining the calibrated measurement value 42. According to examples, knowledge or an assumption that the concentration of the target gas is likely to reach a specific minimum value during a specific time interval, such as one day, may be exploited for revising the baseline value. To this end, according to examples, the information 52 about the further concentration measurement comprises an indication of a minimum concentration obtained by the further device within a time interval, which time interval may be referred to as further time interval, merely to distinguish between the time interval of data gathering of the device 12 and the time interval to which the minimum concentration refers. According to this example, the calibration module 30 uses the indication of the minimum concentration for revising the baseline value. For example, the calibration module 30 may compare the indication of the minimum concentration received from the further device to a minimum concentration determined by the device 12 during a time interval, which may correspond to, but does not necessarily have to correspond to, the further time interval to which the minimum indication of the further device refers. For example, the baseline value may be set in dependence of an offset between the minimum concentration and a concentration value to which the baseline value is associated, e.g., zero. As described before, the confidence of the minimum concentration of the further concentration measurement and/or the confidence of the current calibration of the device 12 may be considered for revising the baseline value.

According to examples, the information about the concentration measurement comprises an indication of a concentration range determined by the further device within a time interval, for example, the further time interval mentioned above, or an even further time interval. For example, the indication on the concentration range may be indicative of a value of a minimum concentration and a value of a maximum concentration measured during the time interval, or may be indicative of an absolute value, such as the minimum concentration, and a difference between a minimum and a maximum value of the concentration measured during the time interval. According to this example, the calibration module 30 revises the calibration information 62 by scaling one or more parameters of the calibration information 62 based on the indication of the concentration range. For example, the one or more parameters to be scaled may include a slope value and/or parameters of a machine learning model, as described before.

Using the information on the minimum concentration and/or the concentration range allows exploiting information of the further device which is not related to the particular time instance at which the devices meet, so that the amount of usable information may be significantly increased. In particular, historic information, as the information on the concentration minimum and/or concentration range of a time interval, may allow for a more accurate compensation of a sensor drift, as it may provide for information on time instances, at which the concentration of the target gas was particularly suitable for recalibration.

It is noted that the revising of the baseline value and the scaling of one or more parameters may be performed independent of each other. For example, the device 12 may differentiate between cases in which the baseline value has drifted and cases in which the sensitivity has changed linearly. In the first case, the recalibration unit 60 may reset the baseline value of the calibration information 62, while in the latter case, the recalibration unit 60 may implement a linear compensation of a calibration curve represented by the calibration information 62, which calibration curve is used for calibrating the measurement information 22. For example, device 12 may detect a baseline drift by comparing a sensor raw signal, e.g., the measurement information 22, at two or more consecutive concentration points, or at two consecutive local minima of a temporal evolution of the raw signal. A change in the sensitivity may, for example, be detected by comparing the range of the raw signal, i.e., a difference between a zero or minimum concentration point or a point in time in which the raw signal is maximum. Additionally or alternatively, minima and/or maxima in the temporal evolution of the raw signal or the calibrated measurement values 42 may be compared to a history of minimum and maximum values received from neighboring devices so as to characterize the drift.

A further approach for revising the calibration information 62, which may, for example, be applied in scenarios in which the drift cannot be clearly characterized, e.g., cannot be clearly attributed to a baseline drift or a drift of sensitivity, or in scenarios in which no reliable neighboring devices were identified at suitable time instances, is the usage of a machine learning model, e.g. a neural network, for processing the information gathered during the time interval of data gathering.

Accordingly, the evaluation of the data collected from the one or more further devices may be accomplished using a neural network which processes the data that was collected, and by mapping the data to an offset estimation for the sensor signal. Using a recurrent neural network or a graph neural network, maybe specifically suitable to process the temporal and spatial dependency of the gathered data. It is noted, however, as described above, that in some cases, in particular, if the neighboring sensor's concentration predictions have a high confidence, simple recalibration techniques may be used, such as the described baseline value recalibration and/or sensitivity recalibration.

Accordingly, in contrast to approaches which rely on algebraic methods or regression models, or in contrast to approaches relying on a single encounter calibration, examples of the present disclosure use the flexibility of machine learning approaches, such as neural networks, in order to address the problem of blind drift calibration with a robust algorithm, that is also applicable in real world applications.

For example, a recurrent neural networks may be employed for analyzing the gathered data gathered, e.g. by successively using the chronologically collected data as an input. Here, the distance may be treated as a normal feature (in contrast to the GNN approach). Alternatively, the data can be assembled as a time-dependent graph, e.g. as shown in Fig. 4. In this case, a graph neural network, GNN, can be used for the analysis of the drift. For example, a data configuration for a GNN graph as illustrated in Fig. 4 may be used.

Accordingly, in examples, the calibration module 30 may check whether the measurement information 22 is subject to a drift type different from one of a drift of the baseline value and a change of the sensitivity, and if so, use a machine learning model, for example a neural network, for revising the calibration information 62 on the basis of the measurement information 22 and the information 52 about the further concentration measurement. For example, this step may be performed after checking whether the measurement information 22 is subject to a drift of the baseline value, and checking whether the measurement information 22 is subject to a change of sensitivity of a sensing unit for providing the measurement information 22. For example, the machine learning model may comprise a neural network such as a recurrent neural network or a graph neural network. By using a recurrent neural network, temporal evolution of the measurement signal may be considered for the recalibration, so that a gradual drift of the measurement information 22 may be accounted for particularly well. The machine learning model may combine all the information gathered during the time interval, including the information on the further concentration measurements and the measurement information 22 and may optionally also consider the time instances associated with the respective measurements, the confidences of the device 12 and the further devices, and optionally information about a distance between the device 12 and the further devices and/or locations of the device 12 and the further devices.

According to examples, the information 52 received from the further device comprises information about a location of the further device. For example, the location may rely on position information or geospatial position information such as GPS or similar. According to this example, the calibration module 30 determines the contribution of the information 52 received from the further device to revising the calibration information 62 in dependence on the location of the further device. For example, device 12 may compare the location of the further device to a location of device 12. Using absolute positions of the device 12 and the further device allows for including further information such as wind direction into the determination of the contribution.

In alternative examples, device 12 is configured for inferring a distance between the device and the further device by evaluating a signal strength of a signal received from the further device via the wireless communication path. According to examples, device 12 may decide, in dependence on whether or not the signal strength is above or below a threshold, or in dependence on whether or not the inferred distance is below a threshold or not, to use the information about the further concentration measurement for revising the calibration information or not. In alternative examples, the calibration module 30 may determine a contribution of the information about the further concentration measurement for revising the calibration information 62 in dependence on the inferred distance. For example, as already described with respect to the consideration of the confidence of the received information, the determination of the contribution may refer to a decision on whether or not to use the received information 52 for revising the calibration information 62, or may refer to setting a weight relative to weights of information received from even further devices and relative to the measurement information 22 of the device 12, which weights determine to which extent the respective information contributes to the revision of the calibration information 62.

A further approach for revising the calibration information 62, which may be used, for example, in scenarios, in which a reference device is available, is revising the calibration information 62 using reference information of the reference device. Accordingly, in examples, device 12 may be configured for checking if a reference device is within a wireless communication range of the device, and if so, revising the calibration information using the reference information of the reference device.

In other words, as described above, the information 52 received from the further device may include one or more or all of a concentration value, for example a current concentration value, determined by the further device, a time stamp indicating a time instance to which the concentration value refers, a location of the further device or a distance between the device 12 and the further device, a prediction confidence indicating a confidence of the gas concentration estimated by the further device, and information about a history of observed minima and maxima in the concentration of the target gas. Optionally the information may further include time stamps of the observed minima and maxima of the concentration.

It is noted that in dependence on a time duration during which the device 12 and the further device are within communication, the amount of data points transmitted between the devices may vary. In other words, in examples, multiple data points, e.g., concentration values, may be transmitted between the device 12 and the further device. More data points for one communication instance can prevent the data from being noisy. Device 12 may buffer the information 52 received from the further device until the data is evaluated.

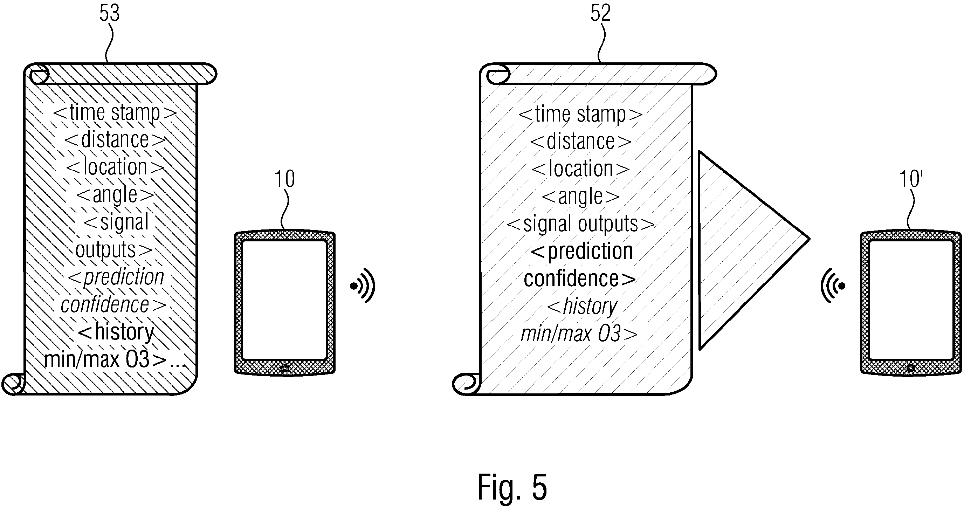

Fig. 5 illustrates an example of an information exchange between mobile device 10 and the further mobile device 10'. The further mobile device 10' provides the information 52 about the further concentration measurement. According to the example of Fig. 5, the information 52 comprises a selection out of a time stamp to which the further concentration measurement refers, a distance between the mobile device 10 and a mobile device 10', a location of the further mobile device 10', an angle at which the further mobile device 10' is oriented in space, signal outputs, a prediction confidence, and a history of minimum and maximum ozone concentrations to which further mobile device 10' was exposed. It is noted that information 52 may, but does not necessarily, comprise all of these information items. For example, 52 may merely comprise one of the distance and the location of the further mobile device. Mobile device 10 may, in turn, provide information 53 about one or more of its own concentration measurements to the further mobile device 10' so that the further mobile device 10' may use the information 53 for recalibrating its own calibration information. For example, information 53 may include the same information items as information 52, however, the information being determined by device 12 of mobile device. In examples, the device 12 may decide whether or not to use the information 52 for recalibrating the calibration information 62 in dependence on whether or not the further mobile device 10' is close enough to the mobile device 10 and/or in dependence on whether the prediction confidence of the further mobile device 10' signaled in information 52 is higher than its own prediction confidence.

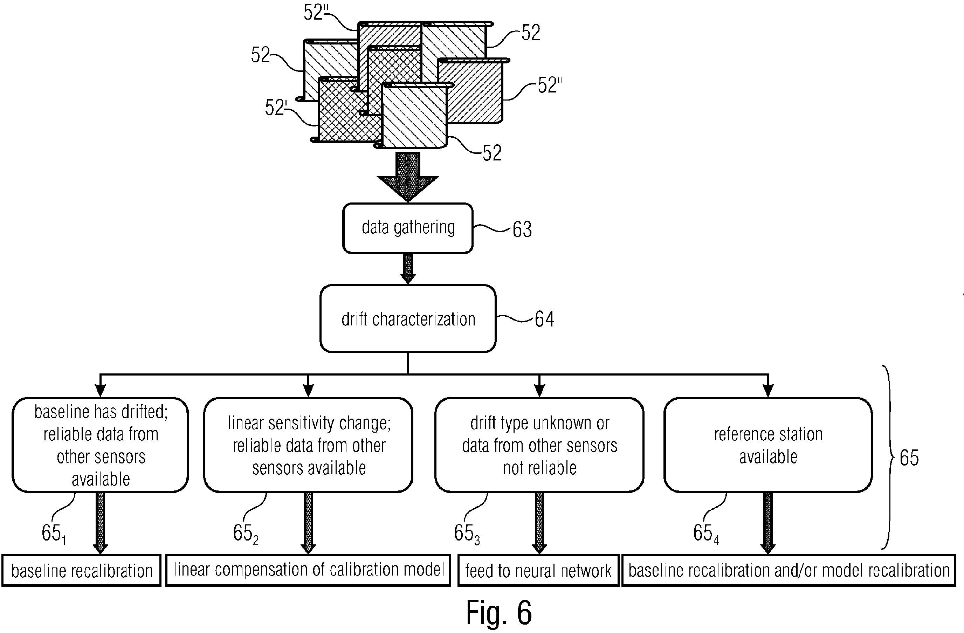

Fig. 6 illustrates an example of an operation scheme of the recalibration unit 60. According to the example of Fig. 6, device 12 gathers information 52, 52', 52" of further mobile devices 10' about respective concentration measurements performed by the mobile devices 10'. Simultaneous to the data gathering phase, the device 12 may optionally monitor its own drifting and non-drifting features extracted from the raw sensor signals. For example, typical drifting features are the sensitivity, e.g., the sensor response normalized to the base line. Examples of non-drifting features are the derivative of the signals or phase and distortion of the first harmonics components of the sensor signal. As described before, device 12 may determine, based on the amount of drift detected, how reliable its current predictions are, i.e., its currently determined calibrated measurement values. The determination of the confidence of the own predictions of device 12 may also be related to the point in time when the last calibration happened, or on an age of the sensing unit providing the measurement information, or on the detection of a defect of the sensing device. For example, device 12 may be capable of detecting and/or diagnosing a fault of the sensing device, e.g. by evaluating a temporal sequence of measurement values and optionally based on information provided by the further device. Device 12 may optionally also use the information, e.g., the concentration values, received from neighboring devices 10', which are located within a predefined distance, for example, by using information on minimum concentration and/or minimum and maximum concentration and/or a concentration range provided by the neighboring devices, as described above.

The data gathering step is referenced using reference sign 63 in Fig. 6 and the step of drift characterization is referenced using reference sign 64. According to the example of Fig. 6, drift characterization 64 is followed by a recalibration step 65, as it may be performed by examples of the recalibration unit 60. Recalibration 65 may comprise one or more of steps 65₁, 65₂, 65₃ and 65₄. Recalibration 65 may be performed in dependence on a type of drift, which has been detected in step 64. For example, if a shift of the baseline has been detected, and if reliable data from other sensing devices is available, steps 65₁ comprising a recalibration of the baseline may be performed, for example, on the basis of a minimum concentration received from the further device as described above. According to step 65₂, if a linear change of the sensitivity is detected, and if reliable data from other sensors is available, a linear compensation of the calibration model is performed. For example, as described above, parameters of the calibration model may be scaled linearly. According to step 65₃, if, according to step 64, an unknown drift type was detected, or if data received from other sensors is not reliable, the received data, and optionally the data determined by a mobile device 10 itself, may be fed into a machine learning model such as a neural network. According to step 65₄, if a reference station is available, a baseline calibration and/or a model recalibration using reference information from the reference station is performed.

For example, step 65₁ may be performed in cases, in which information from a further device is available, which information indicates that the current concentration of the target gas is zero, and which information, according to the indicated confidence, is reliable. In this case, step 65₁ may comprise a recalibration of the baseline value so that current measurement information 22 is mapped to a calibrated measurement value 42 of zero. Step 65₂, that is a compensation by a linear function, may be applied, if a linear drift was detected and the information 52 provided by the further device comprises information about minimum and maximum values for the concentration of the target gas or a concentration range.

Fig. 7 illustrates an example of a gas sensing device 80, as it may optionally be employed in the mobile device 10 and/or the measurement module 20. The gas sensing device 80 may also be referred to as gas sensing unit. The gas sensing device 80 comprises a plurality of sensing units 83, each of which is sensitive to a target gas out of a plurality of target gases. For example, each of the sensing units may be sensitive to a different target gas. Alternatively, one of more of the sensing units 83 may be sensitive to the same target gas, so as to provide redundant measurements signals. It should be noted that one of the sensing units 83 may be sensitive to multiple target gases, wherein the sensitivity of the sensing units 83 may be different for the different target gases. The sensing units 83 may provide respective measurement signals in dependence on concentrations of the target gas to which the sensing units 83 are sensitive. As shown in Fig. 7, but optionally, the gas sensing device 80 may comprise a heater 84, or an individual heater for each of the sensing units 83. During exposure of a sensing unit 83 to a gas, e.g., the target gas, molecules of the case may absorb as a sensing surface of the sensing unit. Heating the sensing surface of the sensing unit 83 may support a desorption of the absorbed gas molecules, preventing a loss of sensitivity of the sensing unit 83.

For example, the gas sensing device 80, which may be referred to as multi-gas sensor array, may consist of four graphene-based, or carbon-based, sensing units 83, in which the base material, e.g., graphene, or another carbon-based material, is functionalized with different chemicals, e.g., Pd, Pt, and Mₙ and O₂, providing for dissimilar selectivity of the four sensing units 83. The interaction between the sensing material, i.e., the functionalized graphene sheets and at absorbed gas analytes would influence the electronic structure of the sensing material, resulting in a modification of the charge carrier density and a modification of the electrical conductances in the sensing materials. Due to the different sensitivities to various gas molecules, the resistances of the sensing units 83 change in disparate patterns, making it possible to analyze complicated gas mixtures with one single sensor array. For example, each of the sensing unit 83 of the array 80 may have a heating element 84 for pulsing the temperature to which the respective sensing unit is exposed, the pulsing beam performed between a recover phase temperature and a sensing phase temperature. Alternatively, the sensing units may be exposed to a sign mode temperature modulation, e.g., for noise reduction using lock in techniques.

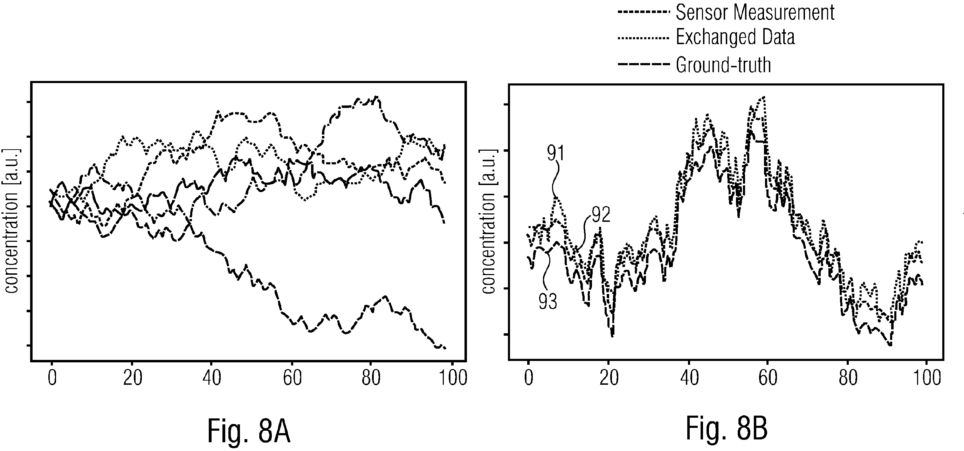

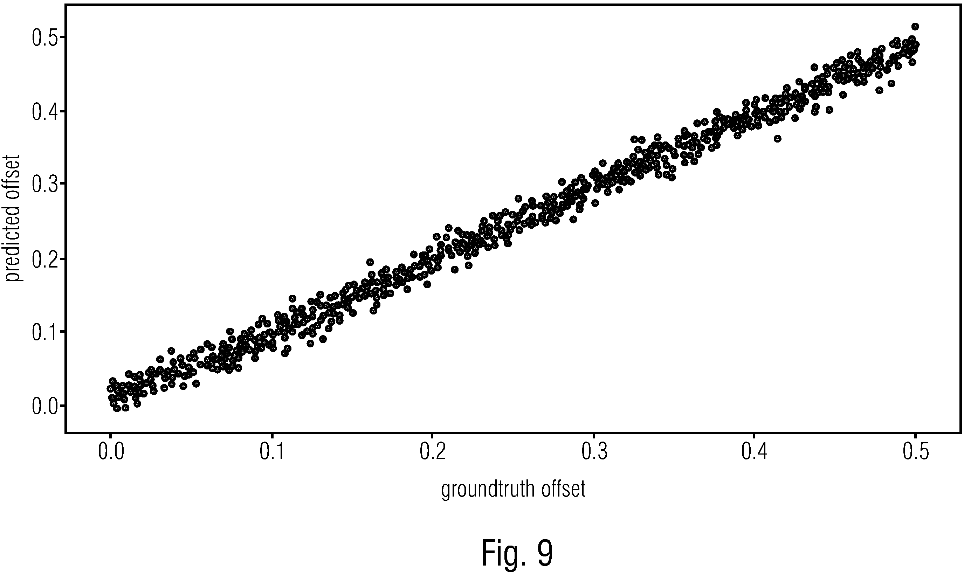

Fig. 8a and Fig. 8b illustrate an evaluation of a simulated test scenario for validating the performance of an example of the disclosed approach. A simulation in a simplified scenario has been conducted in order to test the validity of the approach described above in the case that a Neural Network is applied due to unknown drift behavior. Different potential concentration profiles have been created by implementing a random walk with a *µ* = 0 and *σ* = 0.2. Different such profiles are shown in Figure 8A. The sensor that is about to be calibrated then predicts the concentration profile with a certain randomized offset, which in our simulation is treated as the drift. During the simulation, the sensor comes close to, e.g. encounters, 100 other sensors, where data is exchanged. These sensors are also simulated to have a randomized offset, individually for each encounter, as we expect each sensor to be a different device. The offsets are randomized uniformly between 0 and 0.5. The input features for the algorithm therefore consists of the calibrating sensor measurement at each exchange point, the exchanging sensor's response and a quantized feature assessing the quality of each exchanged data point. This last feature was based on the offset of the exchanging sensor and could take the discrete values {0,1,2}. Fig. 8b shows a plot of the sensor measurement 92, the exchanged data 91 and the ground truth, i.e., the true concentration values, without an offset of one training instance in the supervised training process. As a model, an RNN architecture was chosen, which contains a hidden LSTM layer with 64 neurons, as well as a hidden Dense layer with 10 neurons. The output layer consists of a Dense layer with one output neuron, which corresponds to the estimation of the offset. The model was trained on 800 training samples. In order to test the model, 100 other profiles were simulated, each one enhanced with 10 different offsets. The results in Figure 8 show, that the model was well adjusted to predict the offset with a mean absolute error around 0.0106. The offsets representing the upwards drift were distributed uniformly between 0 and 0.5. Considering these first simulation results of the approach, the proposed calibration method is found to be a promising tool in order to enhance the accuracy of the sensor predictions. Fig. 9 shows a scattered plot showing the ground truth offset and the predicted offset as an output of the model.

For example, the disclosed concept may be applied for stabilization of multi-gas sensors, for example micro-sensors, which are placed at several locations to ensure a high granularity in pollution monitoring. The disclosed method provides an easy and low cost method for maintaining an accurate calibration of such sensors.

As already mentioned, Fig. 2 may also serve as illustration of a method for determining a concentration of a target gas in a mobile application. The method comprises a step 20 of obtaining measurement information about a concentration measurement of the target gas. Further, the method comprises a step 50 of receiving information about a further concentration measurement of the target gas via a wireless communication path. The method further comprises a step 40 of using calibration information for determining a calibrated measurement value 42 on the basis of the measurement information 22, and a step 60 of revising the calibration information by using the received information 52 about the further concentration measurement.

According to an example, the information 52 about the further concentration measurement comprises
- an indication of a further calibrated measurement value of the concentration of the target gas determined by the further device, or an indication of further measurement information obtained by the further device, and
- an indication of a time instance to which the further concentration measurement refers.

According to an example, the information 52 about the further concentration measurement comprises an indication of a confidence of the further concentration measurement, and wherein the calibration module 30 is configured for determining a contribution of the information 52 about the further concentration measurement for revising 60 the calibration information under consideration of the indication of the confidence of the further concentration measurement.

According to an example, the method comprises determining an indication of a confidence of the calibrated measurement value, and revising 60 the calibration information under consideration of the indication of the confidence of the calibrated measurement value.

According to an example, the method comprises determining the indication of the confidence by using one or more of
- a temporal evolution of a sequence of concentration measurements,
- an age of a sensing unit for providing the measurement information,
- an information about a functional state of the sensing unit for providing the measurement information,
- a time instance of a previous revision of the calibration information.

According to an example, the method comprises revising 60 the calibration information using information about a plurality of further concentration measurements received from a plurality of further devices during a time interval.

According to an example, the time interval is between 1 hour and one week, or between 4 hours and 1 week, or between 12 hours and 3 days.

According to an example, the information 52 about the further concentration measurement comprises information about a location of the further device, and the method comprises determining a contribution of the information 52 about the further concentration measurement for revising 60 the calibration information in dependence on the location of the further device.

According to an example, the method comprises inferring a distance between the device and the further device by evaluating a signal strength of a signal received from the further device via the wireless communication path. According to this example, the method comprises using the information 52 about the further concentration measurement for revising 60 the calibration information in dependence on whether the inferred distance is below a threshold, or determining a contribution of the information 52 about the further concentration measurement for revising 60 the calibration information in dependence on the inferred distance.

According to an example, the information 52 about the further concentration measurement comprises an indication of a minimum concentration obtained by the further device within a further time interval, and the method comprises using the indication of the minimum concentration for revising 60 a baseline value comprised in the calibration information, the baseline value being indicative of a constant calibration offset between a measurement value of the measurement information and the calibrated measurement value.

According to an example, the information 52 about the further concentration measurement comprises an indication of a concentration range determined by the further device within a time interval, and the method comprises revising 60 the calibration information by scaling one or more parameters of the calibration information based on the indication of the concentration range.

According to an example, the method comprises using the further measurement information for one or more of
- checking, whether the measurement information is subject to a drift of a baseline value, the baseline value being indicative of a constant calibration offset between a measurement value of the measurement information and the calibrated measurement value,
- checking, whether the measurement information is subject to a change of sensitivity of a sensing unit for providing the measurement information, and
- checking, whether the measurement information is subject to a drift type different from one of a drift of the baseline value and a change of the sensitivity, and if so, using a machine learning model for revising 60 the calibration information on the basis of the measurement information and the information 52 about the further concentration measurement.

According to an example, the method comprises, if a reference device is within a wireless communication range of the device, revising 60 the calibration information using reference information of the reference device.

According to an example, a device 12 for determining a concentration of a target gas in a mobile application comprises a measurement module 20 configured for obtaining measurement information 22 about a concentration measurement of a target gas, a communication module 50 configured for communicating with at least one further device via a direct wireless communication path, wherein the communication module is configured for receiving information 52 about a further concentration measurement of the target gas from the further device, a calibration module 30 configured for using 40 calibration information 62 for determining a calibrated measurement value 42 on the basis of the measurement information 22, and revising 60 the calibration information 62 by using the received information 52 about the further concentration measurement.

According to an example, the information 52 about the further concentration measurement comprises
- an indication of a further calibrated measurement value of the concentration of the target gas determined by the further device, or an indication of further measurement information obtained by the further device, and
- an indication of a time instance to which the further concentration measurement refers.

According to an example, the information 52 about the further concentration measurement comprises an indication of a confidence of the further concentration measurement, and wherein the calibration module 30 is configured for determining a contribution of the information 52 about the further concentration measurement for revising 60 the calibration information under consideration of the indication of the confidence of the further concentration measurement.

According to an example, the device is configured for determining an indication of a confidence of the calibrated measurement value, wherein the calibration module 30 is configured for revising 60 the calibration information under consideration of the indication of the confidence of the calibrated measurement value.

According to an example, the device is configured for determining the indication of the confidence by using one or more of
- a temporal evolution of a sequence of concentration measurements,
- an age of a sensing unit for providing the measurement information,
- an information about a functional state of the sensing unit for providing the measurement information,
- a time instance of a previous revision of the calibration information.

According to an example, the calibration module 30 is configured for revising 60 the calibration information using information about a plurality of further concentration measurements received from a plurality of further devices during a time interval.

According to an example, the time interval is between 1 hour and one week, or between 4 hours and 1 week, or between 12 hours and 3 days.

According to an example, the information 52 about the further concentration measurement comprises information about a location of the further device, and wherein the calibration module 30 is configured for determining a contribution of the information 52 about the further concentration measurement for revising 60 the calibration information in dependence on the location of the further device.

According to an example, the device is configured for inferring a distance between the device and the further device by evaluating a signal strength of a signal received from the further device via the wireless communication path, wherein the calibration module 30 is configured for using the information 52 about the further concentration measurement for revising 60 the calibration information in dependence on whether the inferred distance is below a threshold, or wherein the calibration module 30 is configured for determining a contribution of the information 52 about the further concentration measurement for revising 60 the calibration information in dependence on the inferred distance.

According to an example, the information 52 about the further concentration measurement comprises an indication of a minimum concentration obtained by the further device within a further time interval, wherein the calibration module 30 is configured for using the indication of the minimum concentration for revising 60 a baseline value comprised in the calibration information, the baseline value being indicative of a constant calibration offset between a measurement value of the measurement information and the calibrated measurement value.

According to an example, the information 52 about the further concentration measurement comprises an indication of a concentration range determined by the further device within a time interval, wherein the calibration module 30 is configured for revising 60 the calibration information by scaling one or more parameters of the calibration information based on the indication of the concentration range.

According to an example, the calibration module 30 is configured for using the further measurement information for one or more of checking, whether the measurement information is subject to a drift of a baseline value, the baseline value being indicative of a constant calibration offset between a measurement value of the measurement information and the calibrated measurement value, checking, whether the measurement information is subject to a change of sensitivity of a sensing unit for providing the measurement information, and checking, whether the measurement information is subject to a drift type different from one of a drift of the baseline value and a change of the sensitivity, and if so, using a machine learning model for revising 60 the calibration information on the basis of the measurement information and the information 52 about the further concentration measurement.

According to an example, the device is configured for, if a reference device is within a wireless communication range of the device, revising 60 the calibration information using reference information of the reference device.

According to an example, a mobile apparatus comprises the device according to any of the preceding examples, a gas sensing unit configured for providing the measurement information, and a wireless communication interface as part of the wireless communication path.

According to an example, the at least one further device is part of a further mobile apparatus.

Although some aspects have been described as features in the context of an apparatus it is clear that such a description may also be regarded as a description of corresponding features of a method. Although some aspects have been described as features in the context of a method, it is clear that such a description may also be regarded as a description of corresponding features concerning the functionality of an apparatus.

Some or all of the method steps may be executed by (or using) a hardware apparatus, like for example, a microprocessor, a programmable computer or an electronic circuit. In some examples, one or more of the most important method steps may be executed by such an apparatus.

Depending on certain implementation requirements, examples of the invention can be implemented in hardware or in software or at least partially in hardware or at least partially in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some examples according to the invention comprise a data carrier having electronically readable control signals, which are capable of cooperating with a programmable computer system, such that one of the methods described herein is performed.

Generally, examples of the present invention can be implemented as a computer program product with a program code, the program code being operative for performing one of the methods when the computer program product runs on a computer. The program code may for example be stored on a machine readable carrier.

Other examples comprise the computer program for performing one of the methods described herein, stored on a machine readable carrier.

In other words, an example of the inventive method is, therefore, a computer program having a program code for performing one of the methods described herein, when the computer program runs on a computer.

A further example of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitory.

A further example of the inventive method is, therefore, a data stream or a sequence of signals representing the computer program for performing one of the methods described herein. The data stream or the sequence of signals may for example be configured to be transferred via a data communication connection, for example via the Internet.

A further example comprises a processing means, for example a computer, or a programmable logic device, configured to or adapted to perform one of the methods described herein.

A further example comprises a computer having installed thereon the computer program for performing one of the methods described herein.

A further example according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some examples, a programmable logic device (for example a field programmable gate array) may be used to perform some or all of the functionalities of the methods described herein. In some examples, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer.

The methods described herein may be performed using a hardware apparatus, or using a computer, or using a combination of a hardware apparatus and a computer.

In the foregoing description, it can be seen that various features are grouped together in examples for the purpose of streamlining the disclosure. This method of disclosure is not to be interpreted as reflecting an intention that the claimed examples require more features than are expressly recited in each claim. Rather, as the following claims reflect, subject matter may lie in less than all features of a single disclosed example. Thus the following claims are hereby incorporated into the description, where each claim may stand on its own as a separate example. While each claim may stand on its own as a separate example, it is to be noted that, although a dependent claim may refer in the claims to a specific combination with one or more other claims, other examples may also include a combination of the dependent claim with the subject matter of each other dependent claim or a combination of each feature with other dependent or independent claims. Such combinations are proposed herein unless it is stated that a specific combination is not intended. Furthermore, it is intended to include also features of a claim to any other independent claim even if this claim is not directly made dependent to the independent claim.

The above described examples are merely illustrative for the principles of the present disclosure. It is understood that modifications and variations of the arrangements and the details described herein will be apparent to others skilled in the art. It is the intent, therefore, to be limited only by the scope of the pending patent claims and not by the specific details presented by way of description and explanation of the examples herein.

## Claims

1. Device (12) for determining a concentration of a target gas in a mobile application, the device comprising:
a measurement module (20) configured for obtaining measurement information (22) about a concentration measurement of a target gas,
a communication module (50) configured for communicating with at least one further device via a direct wireless communication path, wherein the communication module is configured for receiving information (52) about a further concentration measurement of the target gas from the further device,
a calibration module (30) configured for
using (40) calibration information (62) for determining a calibrated measurement value (42) on the basis of the measurement information (22), and
revising (60) the calibration information (62) by using the received information (52) about the further concentration measurement.

2. Device (12) according to claim 1, wherein the information (52) about the further concentration measurement comprises
- an indication of a further calibrated measurement value of the concentration of the target gas determined by the further device, or an indication of further measurement information obtained by the further device, and
- an indication of a time instance to which the further concentration measurement refers.

3. Device (12) according to any of the preceding claims, wherein the information (52) about the further concentration measurement comprises an indication of a confidence of the further concentration measurement, and
wherein the calibration module (30) is configured for determining a contribution of the information (52) about the further concentration measurement for revising (60) the calibration information under consideration of the indication of the confidence of the further concentration measurement.

4. Device (12) according to any of the preceding claims, configured for determining an indication of a confidence of the calibrated measurement value,
wherein the calibration module (30) is configured for revising (60) the calibration information under consideration of the indication of the confidence of the calibrated measurement value.

5. Device (12) according to claim 4, configured for determining the indication of the confidence by using one or more of
- a temporal evolution of a sequence of concentration measurements,
- an age of a sensing unit for providing the measurement information,
- an information about a functional state of the sensing unit for providing the measurement information, and
- a time instance of a previous revision of the calibration information.

6. Device (12) according to any of the preceding claims, wherein the calibration module (30) is configured for revising (60) the calibration information using information about a plurality of further concentration measurements received from a plurality of further devices during a time interval.

7. Device (12) according to claim 6, wherein the time interval is between 1 hour and one week, or between 4 hours and 1 week, or between 12 hours and 3 days.

8. Device (12) according to any of the preceding claims, wherein the information (52) about the further concentration measurement comprises information about a location of the further device, and
wherein the calibration module (30) is configured for determining a contribution of the information (52) about the further concentration measurement for revising (60) the calibration information in dependence on the location of the further device.

9. Device (12) according to any of the preceding claims, configured for inferring a distance between the device and the further device by evaluating a signal strength of a signal received from the further device via the wireless communication path,
wherein the calibration module (30) is configured for using the information (52) about the further concentration measurement for revising (60) the calibration information in dependence on whether the inferred distance is below a threshold, or
wherein the calibration module (30) is configured for determining a contribution of the information (52) about the further concentration measurement for revising (60) the calibration information in dependence on the inferred distance.

10. Device (12) according to any of the preceding claims, wherein the information (52) about the further concentration measurement comprises an indication of a minimum concentration obtained by the further device within a further time interval,
wherein the calibration module (30) is configured for using the indication of the minimum concentration for revising (60) a baseline value comprised in the calibration information, the baseline value being indicative of a constant calibration offset between a measurement value of the measurement information and the calibrated measurement value.

11. Device (12) according to any of the preceding claims, wherein the information (52) about the further concentration measurement comprises an indication of a concentration range determined by the further device within a time interval,
wherein the calibration module (30) is configured for revising (60) the calibration information by scaling one or more parameters of the calibration information based on the indication of the concentration range.

12. Device (12) according to any of the preceding claims, wherein the calibration module (30) is configured for using the further measurement information for one or more of
checking, whether the measurement information is subject to a drift of a baseline value, the baseline value being indicative of a constant calibration offset between a measurement value of the measurement information and the calibrated measurement value,
checking, whether the measurement information is subject to a change of sensitivity of a sensing unit for providing the measurement information, and
checking, whether the measurement information is subject to a drift type different from one of a drift of the baseline value and a change of the sensitivity, and if so, using a machine learning model for revising (60) the calibration information on the basis of the measurement information and the information (52) about the further concentration measurement.

13. Device (12) according to any of the preceding claims, configured for, if a reference device is within a wireless communication range of the device, revising (60) the calibration information using reference information of the reference device.

14. Mobile apparatus (10) comprising:
the device (12) according to any of the preceding claims,
a gas sensing unit (80) configured for providing the measurement information, and
a wireless communication interface as part of the wireless communication path.

15. Mobile apparatus (10) according to claim 14, wherein the at least one further device is part of a further mobile apparatus.

16. Method for determining a concentration of a target gas in a mobile application, the method comprising:
obtaining (20) measurement information (22) about a concentration measurement of the target gas,
receiving (50) information (52) about a further concentration measurement of the target gas via a direct wireless communication interface,
using (40) calibration information (62) for determining a calibrated measurement value (42) on the basis of the measurement information, and
revising (60) the calibration information (62) by using the received information (52) about the further concentration measurement.
